(19) Europäisches Patentamt
European Patent Office
Office européen des brevets

(11) Publication number: **0 032 314 A1**

(12) # EUROPEAN PATENT APPLICATION

(21) Application number: 80304710.9

(22) Date of filing: 23.12.80

(51) Int. Cl.[3]: C 07 D 207/333
C 07 D 409/06, A 61 K 31/40

(30) Priority: 09.01.80 GB 8000637

(43) Date of publication of application: 22.07.81 Bulletin 81/29

(84) Designated Contracting States: BE CH DE FR GB LI NL

(71) Applicant: BEECHAM GROUP LIMITED
Beecham House Great West Road
Brentford, Middlesex(GB)

(72) Inventor: Goudie, Alexander Crossan
72 North Brooks
Harlow Essex(GB)

(74) Representative: Dawson, Hugh Bainforde, Dr. et al,
European Patent Attorney BEECHAM
PHARMACEUTICALS Yew Tree Bottom Road
Epsom, Surrey KT18 5XQ.(GB)

(54) **Aroylpyrrole derivatives, processes for their preparation and their use.**

(57) Compounds of the formula (I):

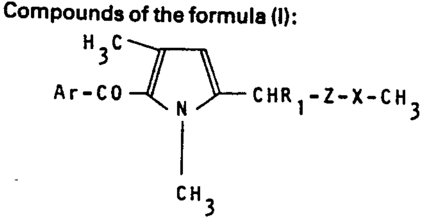

(1)

wherein $R_1$ is a hydrogen atom or methyl group; Ar is a phenyl group or a phenyl group substituted by one or two groups selected from fluorine, chlorine, bromine, methyl, methoxyl or trifluoromethyl or is a thienyl group; X is a CO or CHOH group; Z is a $CH_2$ or CHOH group- and pro drugs thereof having useful pharmacological activity, pharmaceutical compositions containing them and processes for their preparation.

EP 0 032 314 A1

Croydon Printing Company Ltd.

## Aroylpyrrole derivatives, processes for their preparation and their use

The present invention relates to pyrrole derivatives having useful pharmacological activity, to processes for their preparation and to pharmaceutical compositions containing them.

Tolmetin, a clinically used anti-inflammatory and analgesic agent of the formula (A):

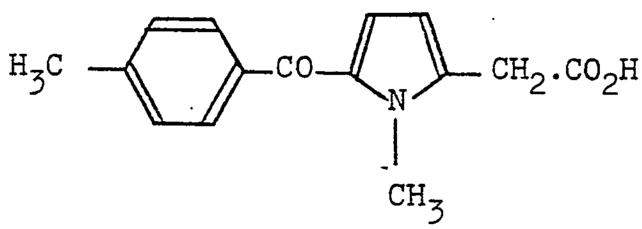

(A)

has been reported in J. Pharmacol, Exptl. Therap. 1973, 185, 127-138 to possess anti-inflammatory activity. Tolmetin and related compounds have also been described in British Patent Specification No: 1195628. It has been found that tolmetin causes gastric irritancy in test animals at doses not greatly exceeding the therapeutic dose. A group of anti-inflammatory and analgesic compounds has now been found which have reduced propensity

to cause gastric irritancy. These compounds may be thus used in pharmaceutical compositions for the treatment of inflammatory or painful conditions such as rheumatism, arthritis, or the like.

The present invention provides the compounds of the formula (I):

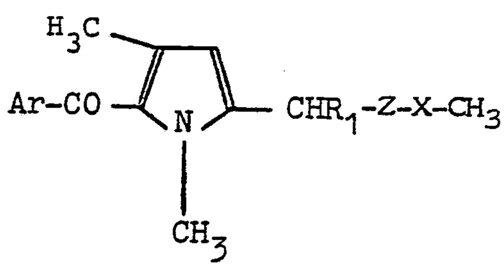

(I)

wherein $R_1$ is a hydrogen atom or methyl group; Ar is a phenyl group or a phenyl group substituted by one or two groups selected from fluorine, chlorine, bromine, methyl, methoxyl or trifluoromethyl or is a thienyl group; X is a CO or CHOH group; Z is a $CH_2$ of CHOH group; and pro-drugs thereof.

A group of compounds within formula (I) include those wherein Z is $CH_2$ and $R_1$, X and Ar are as hereinbefore defined.

Suitable values for Ar include a phenyl or substituted phenyl group.

More suitably Ar is a phenyl or mono-substituted phenyl group.

Particularly apt groups Ar include the phenyl methylphenyl, trifluoromethyl-phenyl, chlorophenyl and the methoxyphenyl group.

A favoured group Ar is the phenyl group. A further

favoured group Ar is the 4-methylphenyl. Another favoured group Ar is the 4-chlorophenyl group. Yet a further favoured group Ar is the 4-fluorophenyl group. Yet another favoured group Ar is the 4-methoxyphenyl group.

Other suitable values for Ar include di-halogenated phenyl such as di-chlorophenyl, for example 2,4-dichlorophenyl.

Further suitable values for Ar include a thienyl group. Another favoured group Ar is the 2-thienyl group. A further favoured group Ar is the 3-thienyl group.

Preferably Ar is a 4-methylphenyl or 4-chlorophenyl group.

Suitably $R_1$ in the preceding compounds represents a hydrogen atom.

Suitably $R_1$ in the preceding compounds represents a methyl group. Preferably $R_1$ is hydrogen.

Suitably Z in the preceding compounds is a CHOH group.

Suitably Z in the preceding compounds is a $CH_2$ group.

Preferably Z is a $CH_2$ group.

Suitably X in the preceding compounds is a CO group.

Suitably X in the preceding compounds is a CHOH group.

Preferably X is a CO group.

When used herein the term 'pro-drug' means a compound metabolised <u>in-vivo</u> to or via a compound of the formula (I).

The pro-drugs will be derivatives of the group X. Examples of pro-drugs of compounds wherein Z is $CH_2$ are those wherein the 2- position side chain ie the $CHR_1$-Z-X-$CH_3$ group is replaced by a group of the sub-formulae (a) - (d);

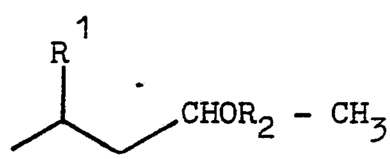

(a)

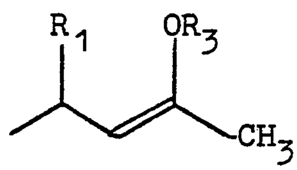

(b)

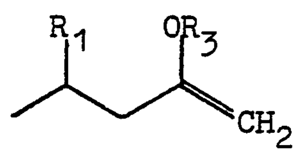

(c)

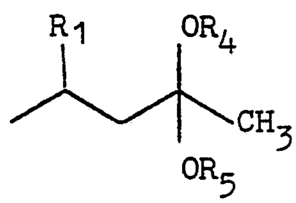

(d)

wherein $R_1$ is a hydrogen atom or a methyl group; $R_2$ is a group CO.$R_6$ wherein $R_6$ is the residue of a pharmaceutically acceptable carboxylic acid of up to 9 carbon atoms of the formula $R_6COOH$; $R_3$ is a $C_{1-4}$ alkyl group or a CO.$R_6$ group; $R_4$ is a methyl, ethyl or propyl group and $R_5$ is a methyl, ethyl or propyl group or $R_5$ is joined to $R_4$ so that they together represent a $CH_2CH_2$ or $CH_2CH_2CH_2$ group.

A favoured 2- position side chain in the preceding compounds of formula (I) is the $CH_2.CH_2.CO.CH_3$ group.

Another favoured 2- postion side chain in the preceding compounds of formula (I) is the $CH_2.CH_2.CHOH.CH_3$ group.

Further favoured 2- position side chains are those of the formula $CH_2.CH_2.CH(O.CO.R_6)CH_3$ wherein $R_6$ is as defined in relation to sub-formula (a).

Apt values for $R_6$ include phenyl, alkyl of 1-4 carbon atoms, and alkyl of 1-4 carbon atoms substituted by phenyl, or one of the aforementioned groups substituted by a hydroxyl, acetoxyl, methoxyl, acetamido, optionally salted amino or alkylamino or optionally salted carboxyl group.

Favoured values for $R_6$ include the methyl, ethyl, n-propyl, iso-propyl, t-butyl, phenyl, benzyl, phenylethyl, acetoxymethyl, methoxymethyl, hydroxymethyl optionally salted aminoethyl, α-acetoxyphenyl, 4-methoxyphenyl, 3,4-dimethoxyphenyl and 3,4,5-trimethoxyphenyl groups.

Particularly suitable values for $R_6$ include the methyl, ethyl, benzyl, 2-methoxyphenyl, phenyl and 3,4,5-trimethoxyphenyl group.

A preferred group $R_6$ is the methyl group.

From the foregoing it will be realised a further favoured 2- postion side-chain is the $CH_2.CH_2.CH(O.CO.CH_3)CH_3$ group.

One group of favoured 2-position side chains is that of the formula $CH_2CH_2X^1CH_3$ where $X^1$ is a CO, CHOH or $CHOCOR_7$ group where $R_7$ is an alkyl group of 1-4 carbon atoms. Most suitably $R_7$ is a methyl group.

The compounds of formula (I) are capable of existing in a number of stereoisomeric forms. The invention extends to each of these stereoisomeric forms and to mixtures thereof (including racemates). The different isomeric forms may be separated one from the other by conventional methods.

Certain particularly effective compounds of this invention include those of the formula (III):

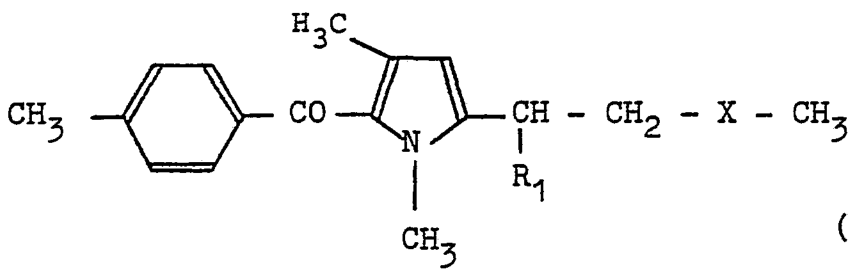

(III)

wherein $R_1$ is a hydrogen atom or methyl group and X is a CO or CHOH group.

In the compounds of formula (III) $R_1$ is suitably a hydrogen atom. In the compounds of the formula (III) $R_1$ is suitably a methyl group. Preferably $R_1$ is a hydrogen atom.

In the compounds of the formula (III) X is suitably a CO group. In the compounds of the formula (III) X is suitably a CHOH group. Preferably X is a CO group.

Certain other particularly effective compounds of the invention include those of the formula (IV):

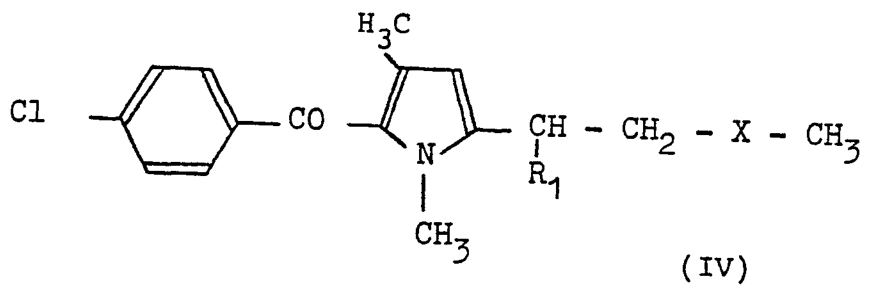

(IV)

wherein $R_1$ is a hydrogen atom or a methyl group and X is a CO, or CHOH group.

In the compounds of the formula (IV) $R_1$ is suitably a hydrogen atom. In the compounds of the formula (V) $R_1$ is suitably a methyl group. Preferably $R_1$ is a hydrogen atom.

Suitably in the compounds of the formula (IV) X is a CO group. Suitably in the compounds of the formula (IV) X is a CHOH group. Preferably X is a CO group.

Certain further particularly effective compounds of this invention include those of the formula (V):

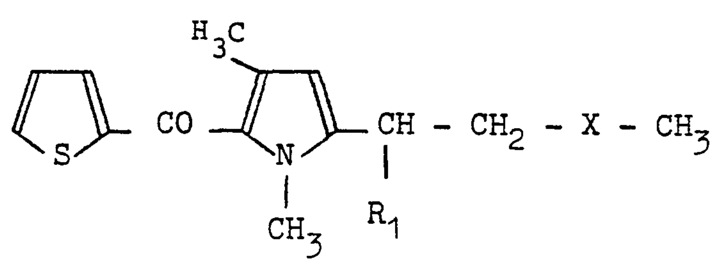

(V)

wherein $R_1$ is a hydrogen atom or a methyl group and $X^1$ is a CO or CHOH group.

In the compounds of the formula (V) $R_1$ is suitably a hydrogen atom. In the compounds of the formula (V) $R_1$ is suitably a methyl group. Preferably $R_1$ is a hydrogen atom.

In the compounds of the formula (V) X is suitably a CO group. In the compounds of the formula (V) X is suitably a CHOH group. Preferably X is a CO group.

A group of compounds of interest within formula (I) are of formula (VI) and pro-drugs thereof:

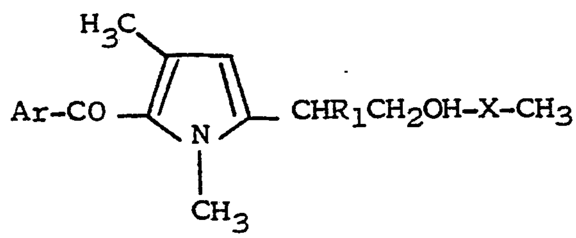

(VI)

wherein $R_1$, X and Ar are as defined in formula (I). Suitable and preferred values for $R_1$, X and Ar are as hereinbefore described under formula (I).

Suitable compounds within formula (VI) include those of formula (VII):

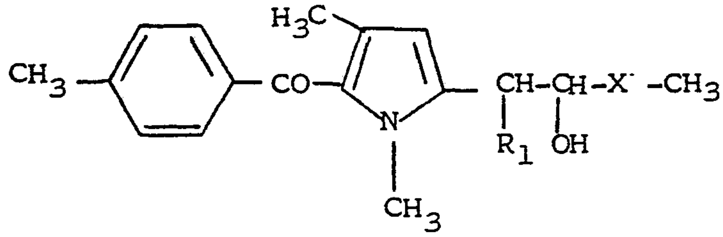

(VII)

wherein

$R_1$ is as defined in formula (I); and

$X^1$ is as defined in formula (III).

Suitable and preferred values for X and $R_1$ are as described under formula (III).

Other suitable compounds within formula (VI) include those of formula (VIII):

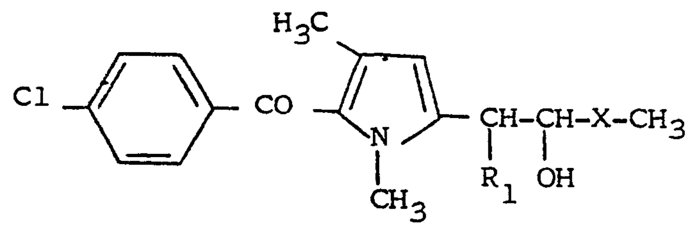

(VIII)

wherein

$R_1$ is as defined in formula (I); and

X is as defined in formula (III)

Suitable and preferred values for $R_1$ and X are as described under formula (III).

Particularly suitable compounds of this invention include:

4-(1,4-dimethyl-5-p-toluoyl-2-pyrryl)butan-2-one;
2-acetoxy-4-(1,4-dimethyl-5-p-toluoyl -2-pyrryl)butane;
4-(1,4-dimethyl-p-chlorobenzoyl-2-pyrryl)butan-2-one;
4-(1,4-dimethyl-p-chlorobenzoyl-2-pyrryl)butan-2-ol;
2-acetoxy-4-(1,4-dimethyl-5-p-chlorobenzoyl-2-pyrryl)butane;
4-(1,4-dimethyl-5-thien-2'-oyl-2-pyrryl)butan-2-one;
4-(1,4-dimethyl-5-thien-2'-oyl-2-pyrryl)butan-2-ol;
2-acetoxy-4-(1,4-dimethyl-5-thien-2'-oyl-2-pyrryl)-butane;
4-(1,4-dimethyl-5-p-tolyoyl-2-pyrryl)butan-2-ol;

4-(1,4-dimethyl-5-p-toluoyl-2-pyrryl)-3-hydroxy-butan-2-one;

4-(1,4-dimethyl-p-chlorobenzoyl-2-pyrryl)-3-hydroxy-butan-2-one;

4-(1,4-dimethyl-p-chlorobenzoyl-2-pyrryl)butan-2,3-diol;

4-(1,4-dimethyl-5-p-toluoyl-2-pyrryl)butan-2,3-diol.

In a further aspect this invention provides a pharmaceutical composition which comprises a compound of the formula (I) or pro-drug thereof and a pharmaceutically acceptable carrier.

The compositions of this invention are useful in treating rheumatic and arthritic conditions because of their anti-inflammatory and analgesic properties. The compositions may be adapted for administration via the oral, rectal or injection routes but since the compositions of this invention do not excessively irritate the gastro-intestinal tract it is preferred that they are adapted for oral administration.

The compositions of this invention may contain diluents, binders, fillers, disintegrants, flavouring agents, colouring agents, lubricants, preservatives or the like in conventional manner. These conventional excipients may be employed in conventional manner, for example as in the preparation of compositions of ketoprofen, indomethacin, naproxen, acetylsalicylic acid or other anti-inflammatory analgesic agents.

Most suitably the composition of this invention will be in the form of a unit dose such as a tablet,

capsule or reconstitutable powder in a sachet. Such unit doses will generally contain from 20 mg to 1000 mg and more suitably will contain from about 30 mg to 500 mg for example 50 mg to 250 mg of active agent, for example about 50, 100, 150, 200, 250, 300, 350, 400, 450 or 500 mg. These compositions may be administered once or more times a day, for example 2, 3 or 4 times daily, so that the total daily dose for a 70 Kg adult will usually be in the range 80 to 4000 mg and more usually in the range 300 to 3000 mg for example 500 to 2000 mg.

A favoured form of the composition of this invention is a hard gelatin capsule containing the active agent. The active agent may be in the form of a powder, granulate or the like and may advantageously be in intimate mixture with a lubricant such as magnesium stearate.

A further favoured form of the composition of this invention is a tablet containing the active agent. The active agent may be in the form of a recompressed granulate of the active ingredient in intimate mixture with a lubricant such as magnesium stearate, a filler such as microcrystalline cellulose and a disintegrant such as sodium starch glycollate.

The present invention also provides a method of treating inflammatory and/or painful conditions in mammals which comprises administering per day from 200 to 4000 mg of a compound of this invention and more usually from 300 to 3000 mg for example from 500 to 2000 mg of a compound of this invention.

Mammals which may be thus treated include humans and domestic animals such as dogs, cats or horses.

Most suitably the medicament will be administered orally as 2, 3 or 4 doses per day at the dose level previously indicated.

Often the condition treated will be arthritis.

The present invention provides a process for the preparation of a compound of the formula (I) wherein Z is $CH_2$, or a pro-drug thereof which process comprises the reaction of a compound of the formula (IX):

Ar.CO.Cl (IX)

or an equivalent acylating agent wherein Ar is as defined in relation to formula (I), with a compound of the formula (X):

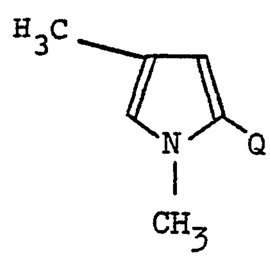

(X)

wherein Q is a group of the sub-formulae (a) - (d) as hereinbefore defined or a group of the sub-formula (e):

$$-CHR_1-CH_2-CO-CH_3 \qquad (e)$$

wherein $R_1$ is a hydrogen atom or a methyl group; and thereafter if desired or necessary reducing the carbonyl present in a group of the sub-formula (e) to a CHOH group.

The present invention also provides a process for the preparation of the compounds of the formula (I) wherein Z is $CH_2$ which process comprises the reaction of a compound of the formula(IX):

Ar.CO.Cl (IX)

or an equivalent acylating agent wherein Ar is as defined in relation to formula (II), with a compound of the formula (XI):

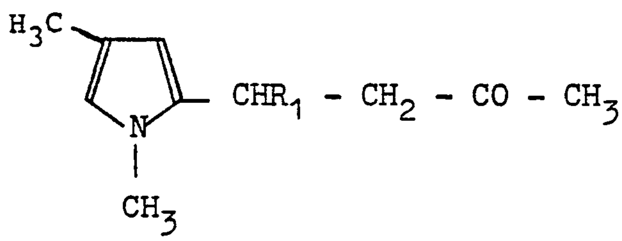

(XI)

wherein $R_1$ is as defined in relation to formula (I) and thereafter if desired reducing the carbonyl group X to a CHOH group X and/or thereafter converting the CO or CHOH group X to a pro-drug thereof.

The present invention also provides a process for the preparation of the pro-drugs of the compounds of the formula (I) wherein Z is $CH_2$ which process comprises the reaction of a compound of the formula (IX) as hereinbefore defined or an equivalent acylating agent with a compound of the formula (XII):

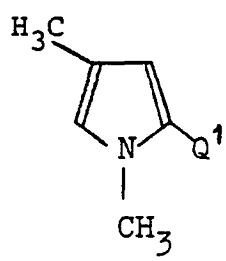

(XII)

wherein $Q^1$ is a group of the sub-formulae (a) - (d) as hereinbefore defined.

Suitable equivalent acylating agents to the compounds of the formula (IX) include the corresponding bromide, anhydride and the like, for example the corresponding azide or mixed anhydride.

The reaction of the compounds of the formulae (IX) and (X), (XI) or (XII) takes place in an inert solvent or under conventional Friedel-Crafts acylation conditions, for example in an inert solvent and optionally in the presence of a Lewis acid such as aluminium chloride.

The acylation reaction is normally carried out at a non-extreme temperature for example from about 5°C to 50°C and more usually from about 10°C to 30°C if a Lewis acid is used. If no catalyst is used the acylation reaction is normally carried out at a higher temperature e.g. 100°C.

Suitable solvents for carrying out the acylation include tetrachloroethylene, chloroform, dichloromethane, dichloroethane, chlorobenzene or the like or benzene, toluene, nitrobenzene or the like.

The solvent system used for the process of this invention will be homogenous and will advantageously comprise an inert component and a tertiary amine. In general the inert component will predominate, for example it will comprise 60%-90% v/v of the total system and more usually from 80% to 92% v/v. Toluene and tetrachloroethylene are favoured inert solvents. Suitable tertiary amines include conventional weak tertiary amines such as pyridine and the like.

When the solvent system employed contains a tertiary amine it is frequently advantageous not to employ a Lewis acid catalyst as acceptable yields are obtained in the absence of said catalyst. This form of the reaction may be performed at a low, ambient or elevated temperature but in general it is preferred to use

a somewhat elevated temperature to ensure that the reaction is over in a reasonably short period. Thus, for example, a temperature of 40-140°C is generally suitable, for example 80-120°C.

The product produced by acylation in the presence of a Lewis acid may be isolated in conventional manner, for example by diluting with an aqueous acid, extracting into an organic solvent, washing and drying the organic phase and thereafter evaporating the solvent. The resulting diketone may then be purified by chromatography and/or recrystallisation.

The product produced by acylation in the absence of a Lewis acid may often be obtained simply by the evaporation of the solvents. If the resulting product is required in a purer form it may normally be further purified by chromatography in conventional manner.

The diketones of the formula (I) (ie when X is CO and Z is $CH_2$) may be converted to the corresponding compounds wherein X is a CHOH by careful reduction with a complex hydride such as sodium borohydride in an lower alcohol. The resulting compound may be separated by conventional methods of column chromatography from any contaminant resulting from reduction of the aromatic ketone.

The compounds wherein X is a CHOH group may be acylated in conventional manner, for example, by reaction with the acid $R_2CO_2H$ in the presence of a condensation promoting agent such as dicyclohexylcarbodiimide in an aprotic solvent such as dichloromethane or tetrahydrofuran or by reaction with an acyl halide in the presence of an acid acceptor such as pyridine.

The pro-drugs of the compounds of the formula (I) may be prepared from the compounds of the formula (I) in conventional manner.

Thus, for example, those compounds containing a side chain of the sub-formula (a) may be prepared by the acylation of a compound of formula (I) containing a 2-position side chain of the sub-formula (f):

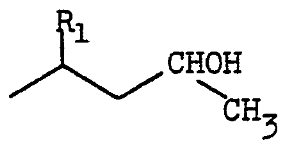

(f)

Acylation may be carried out by conventional methods such as those described in U.K. Patent No. 1538473.

Also for example, those compounds containing a side chain of the sub-formulae (b), (c) or (d) may be prepared by the enol acylation, enol etherification or acetalation of a compound of formula (I) containg a 2-postion side chain of the sub-formula (f):

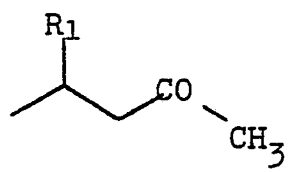

(e)

Suitable conventional methods of enol acylation, enol etherification or acetalation include those described in U.S. Patent No. 4180585.

The present invention also provides a process for the preparation of a compound of the formula (I) wherein X is a CO group and Z is $CH_2$ which process comprises the oxidation of a compound of the formula (XIII):

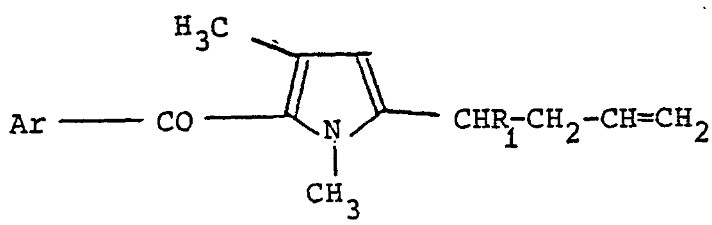

(XIII)

wherein Ar and $R_1$ are as defined in relation to formula (I) for example, with oxygen in aqueous dimethylformamide in the presence of palladium chloride and cuprous chloride.

The oxidation reaction may be effected using pure oxygen or air. In general it is sufficient to blow air through the reaction mixture at an ambient or slightly elevated temperature to effect oxidation. The desired compound may be obtained from the reaction mixture by dilution with water followed by extraction into water-immiscible solvent such as chloroform which may then be dried and evaporated. This initial crude material may be purified chromatographically if desired, for example by column chromatography over silica gel using 1:1 ether : petrol as eluant.

The compounds of the formula (XIII) may be prepared by the decarboxylation of a corresponding compound of the formula (XIV):

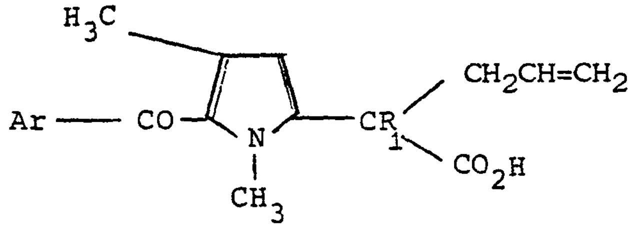

(XIV)

wherein Ar and $R_1$ are as defined in relation to formula (IV).

The decarboxylation may be effected by heating, for example to 170 - 210°C. The desired product may be obtained by trituration under a non-hydroxylic solvent such as chloroform.

The acid of the formula (XIV) may be obtained by hydrolysis of the corresponding ethyl ester using normal sodium hydroxide solution followed by neutralisation with hydrochloric acid.

The desired ethyl ester may be prepared by the allylation of the corresponding compound of the formula (XV):

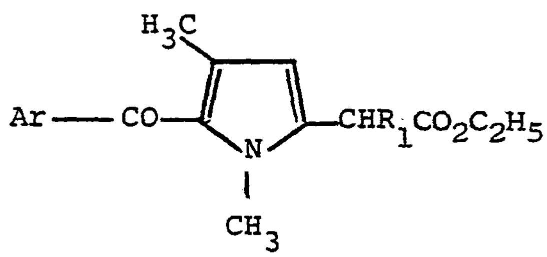

(XV)

wherein Ar and $R_1$ are as defined in relation to formula (I). Such allylations may be brought about by generating an anion of the formula (XV), for example with sodium hydride in dimethoxyethane, and quenching said anion with allyl bromide.

Alternatively, compounds of the formula (XIV) may be prepared by direct allylation of the carboxylic acid derived from a compound of formula (XV). The ethyl ester of a compound of the formula (XIV) wherein $R_1$ is a methyl group may alternatively be prepared by methylation of the corresponding ethyl ester wherein $R_1$ is a hydrogen atom, for example by sequential reaction with sodium hydride and methyl iodide.

The present invention also provides a process for the preparation of a compound of the formula (I) wherein Z is CHOH which process comprises the oxidation of a compound of the formula (I) wherein the 2-position side chain is of the formula (b) wherein $R_3$ is a $C_{1-4}$ alkyl group, that is a compound of formula (XVI):

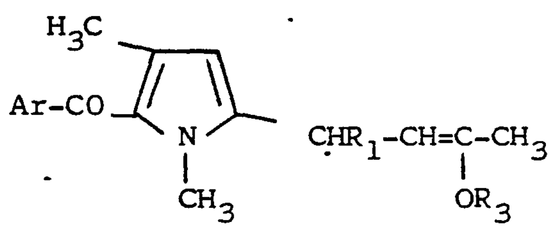

(XVI)

and thereafter if desired or necessary reducing the resulting compound of formula (I) wherein Z is CHOH and X is CO to a compound wherein Z is CHOH and X is CHOH.

The oxidation is suitably carried out using m-chloroperbenzoic acid at 0-5°C in mixed solvents such as diethylether/water.

Reduction of compounds wherein X is CO may be effected using a complex hydride such as sodium borhydride under conventional conditions.

Compounds of the formula (XVI) may be prepared by enol etherification of a compound of formula (I) containing a 2-position side chain of the sub-formula (e) as hereinbefore defined.

The following Example illustrates the invention and the following description illustrates the preparation of intermediates thereto.

Description 1

Reaction of Ethyl 1,4-dimethyl-5(p-chlorobenzoyl)-pyrrole-2-acetate with allyl bromide.

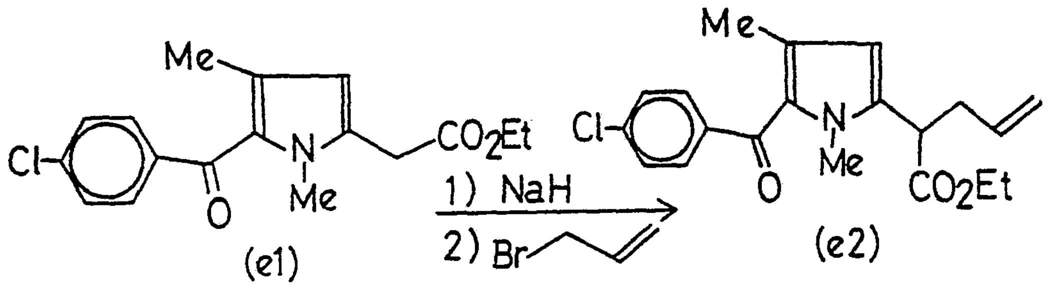


(e1) (e2)

A suspension of the ester (e1) (2.5 g, 0.00867 mole) in dimethoxyethane (about 10 ml) was added to a stirred slurry of 55% sodium hydride (0.38 g, 1 equivs) in dimethoxyethane (12 ml) under nitrogen. The mixture was stirred for 5h before adding allyl bromide (1.05 g, 1 equivs) dropwise. A slow decrease in intensity of the red colouration was observed. After stirring at room temperature overnight the solution contained a precipitate (yellow/brown). The solution was poured into ice/dil HCl, extracted with chloroform, the organic layers washed with water, dried (Mg $SO_4$) and evaporated to dryness to give (e 2) as an orange oil (3.3 g).

N.M.R.: $\tau$($CDCl_3$) 2.3 (d-2H, J = 9 Hz), 2.58 (d-2H, J = 9Hz), 3.97 (s-1H), 4.05 - 5.1 (multiplets - 4H), 5.77 (q - 2H), 6.2 (s - 3H), 7.22 (M - 2H), 8.24 (s - 3H), 8.72 (t - 3H).

Description 2

Hydrolysis of the crude reaction product from Description 1

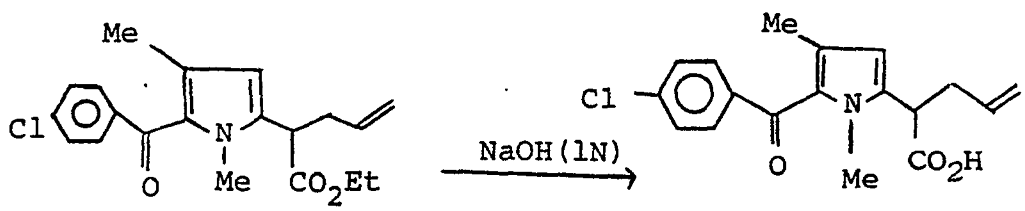


(e3)

The crude ester (e 2) (3.1g, 0.0086 mole) was refluxed for 45 mins. with 1N NaOH (11 ml). After extraction with chloroform followed by ether the diluted aqueous solution was heated for about 10 mins. with animal charcoal and then filtered to give a yellow-green solution (slightly cloudy) This was refiltered through a plug of Kieselguhr and acidified with dilute HCl to give a yellowish emulsion which slowly coagulated. After collection by filtration and drying (e3) as a yellow solid (1.17g) was obtained.

N.M.R. : τ ( $(CD_3)_2SO$): 2.39 (s-4H), 3.95 (s-1H), 4.0 - 5.1 (multiplets - 4H), 6.22 (s-3H), 7.3 (m-2H), 8.29 (s-3H).

Description 3

Pyrolysis of Acid

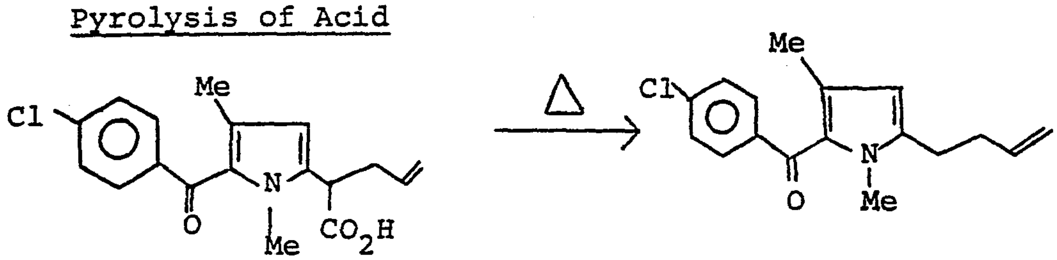


(e3) (e4)

a) Small Scale

The acid (e3) (215 mg) was heated at 180-205° under nitrogen for about 25 mins. A gas was evolved and a dark brown oil was formed. This was dissolved in chloroform, filtered and evaporated to dryness to give an orange oil (200 mg) containing (e4).

b) Larger Scale

The acid (e3) (800 mg) was pyrolyzed as above at 175-190° for 15 mins. Evolution of gas had ceased after 7 mins. Work up as in a) gave (e4) as an orange oil (739 mg) which solidified on standing.

Column chromatography (silica gel 50g eluted with 3 : 1 petrol:ether at first, then progressively more ether. ( max of 2:1 petrol ether) gave pure (e4) from the first fractions (0.38 g). Later fractions gave a mixture (0.24 g) of which the major component was the terminal olefin (mp 78-80°C).

n.m.r.

τ ($CDCl_3$): 2.36 (d-2H, Je 9Hz), 2.64 (d-2H, J = 9Hz), 3.7-4.5 [(m-1H) and overlapping (s-1H) at 4.28], 4.7 - 5.2 [multiplets - 2H], 6.28 (S-3H), 7.44 (m-4H), 8.28 (S-3H).

| Analysis | | Calc. | Found |
|---|---|---|---|
| | C | 70.95 | 70.99 |
| $C_{17}H_{18}ClNO$: | H | 6.30 | 6.46 |
| | N | 4.87 | 4.86 |
| | Cl | 12.32 | 12.31 |

Example 1

Reaction of 4-(1,4-dimethyl-5-p-chlorobenzoyl-2-pyrryl) butene with palladium chloride/copper (I) chloride/$H_2O$

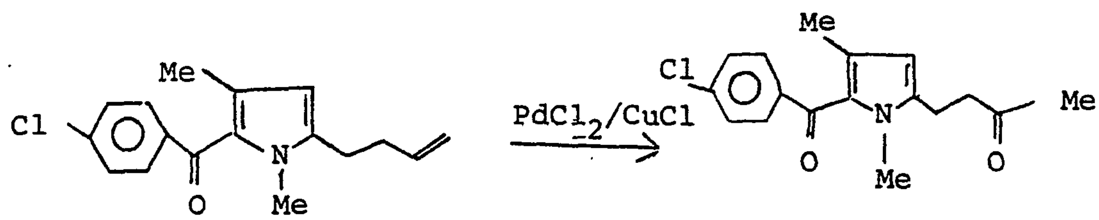


(e4) (e5)

Copper (I) chloride (83 mg) and palladium chloride (30 mg) were added to water (0.1ml) in DMF (1ml) to give a black suspension. Air was blown through this mixture for 1½ hours with shaking before adding (e4) (0.24 g 0.00084 mole). After warming briefly under hot water tap to effect solution a green colouration was obtained The mixture was again shaken while blowing through with air.

The reaction mixture was worked up after 1½ hours by pouring into water (25 ml) and extracting with chloroform (x3). The combined extracts were washed with water (x4) dried and evaporated to dryness to give a yellow oil (0.24 g)

Column chromatography (silica gel (20 g) eluted with 1:1 ether:petrol) gave a yellow solid which was recrystallised from ether to give yellow needles of (e5), melting point 115-118°.

n.m.r. $\tau(CDCl_3)$: 2.27 (d-2H, J=9Hz), 2.55 (d-2H, J=9Hz), 4.2 (5-1H), 6.23 (5-3H), 7.16 (S-4H), 7.78 (S-3H), 8.27 (S-3H).

i.r. 1710(s), 1600(s)

Pharmacological Data

Analgesic activity was determined using a conventional phenylquinone induced mouse writhing test.

The compound of example 1 had an $ED_{50}$ of 6.1 mg/kg p.o.

Toxicity

No toxic effects were observed in the above test.

Claims

1. A compound of formula (I):

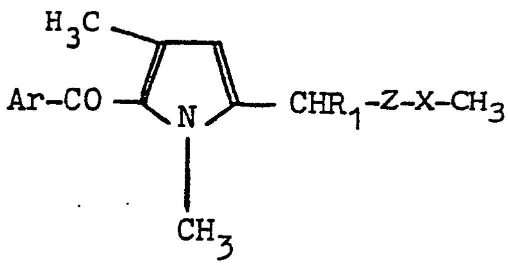

(I)

wherein $R_1$ is a hydrogen atom or methyl group; Ar is a phenyl group or a phenyl group substituted by one or two groups selected from fluorine, chlorine, bromine, methyl, methoxyl or trifluoromethyl or is a thienyl group; X is a CO or CHOH group; Z is a $CH_2$ or CHOH group; or a pro-drug thereof.

2. A compound according to claim 1 wherein Z is $CH_2$.

3. A compound according to claims 1 or 2 wherein Ar is a phenyl group or a substituted phenyl group.

4. A compound according to claim 3 wherein Ar is a 4-methylphenyl or 4-chlorophenyl group.

5. A compound according to claim 1 of the formula (III):

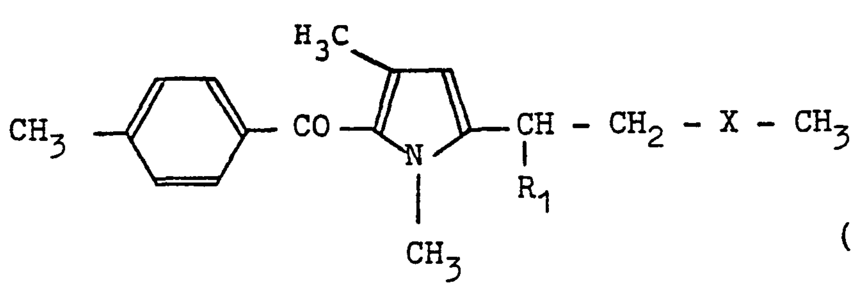

(III)

wherein $R_1$ is a hydrogen atom or methyl group and X is a CO or CHOH group.

6. A compound according to claim 1 of the formula (IV):

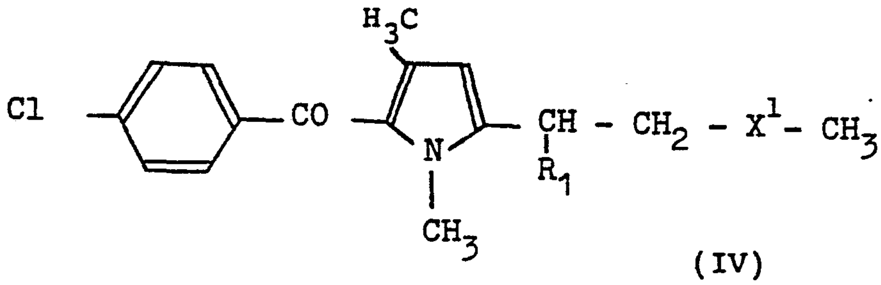

(IV)

wherein $R_1$ is a hydrogen atom or a methyl group and X is a CO or CHOH group.

7. A compound according to any one of claims 1 to 6 wherein X is CO.

8. A compound according to any one of the claims 1 to 7 wherein $R_1$ is hydrogen.

9. 4-(1,4-Dimethyl-5-p-toluoyl-2-pyrryl)butan-2-one, or 4-(1,4-dimethyl-p-chlorobenzoyl-2-pyrryl)butan-2-one.

10. A pro-drug of a compound according to claim 2, wherein the 2-position side chain is replaced by a group of sub-formulae (a)-(d):

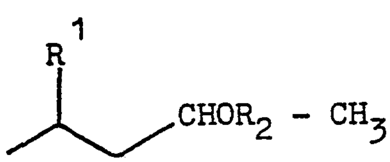

(a)

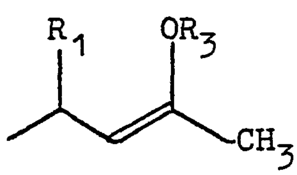

(b)

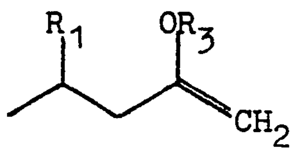

(c)

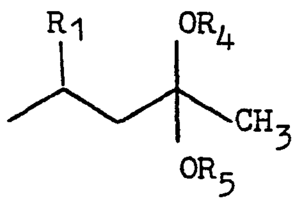

(d)

wherein $R_1$ is a hydrogen atom or a methyl group; $R_2$ is a group $CO.R_6$ wherein $R_6$ is the residue of a pharmaceutically acceptable carboxylic acid of up to 9 carbon atoms of the formula $R_6COOH$; $R_3$ is a $C_{1-4}$ alkyl group of a $CO.R_6$ group; $R_4$ is a methyl, ethyl or propyl group and $R_5$ is a methyl, ethyl or propyl group or $R_5$ is joined to $R_4$ so that they together represent a $CH_2CH_2$ or $CH_2CH_2CH_2$ group.

11. A process for the preparation of a compound according to claim 1 characterised by

(i) when Z is $CH_2$:

(a) reacting a compound of the formula (IX):

$$Ar.CO.Cl \qquad (IX)$$

or an equivalent acylating agent wherein Ar is as defined in relation to claim 1, with a compound of the formula (X):

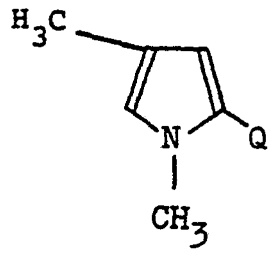

(X)

wherein Q is a group of the sub-formulae (a)-(d) as hereinbefore defined or a group of the sub-formula (e):

$$-CHR_1-CH_2-CO-CH_3 \qquad (e)$$

wherein $R_1$ is a hydrogen atom or a methyl group; and thereafter is desired or necessary reducing the carbonyl present in a group of the sub-formula (e) to a CHOH group.

or (b) oxidising a compound of formula (XIII):

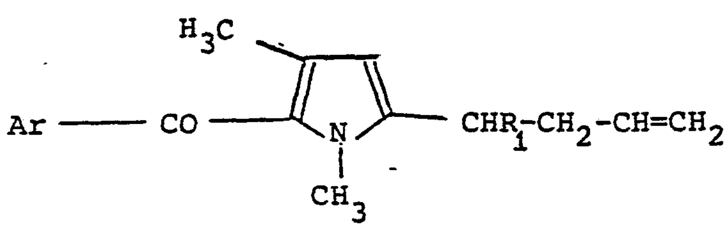

(XIII)

wherein Ar and $R_1$ are as defined in relation to claim 1.

(ii) when Z is CHOH, the oxidation of a compound of formula (XVI):

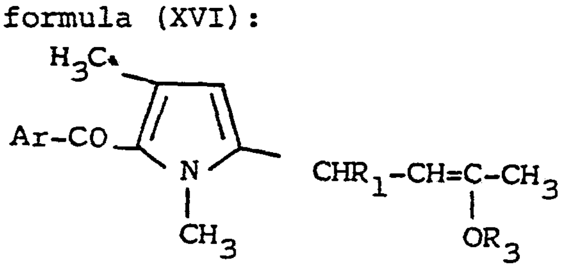

(XVI)

wherein Ar and $R_1$ are as defined in claim 1 and $R_3$ is as defined in claim 10.

12. A process for the preparation of a pro-drug according to claim 10 characterised by reacting a compound of formula (IX) as defined in claim 11 or an equivalent acylating agent with a compound of the formula (XII):

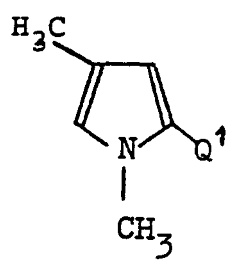

(XII)

wherein $Q^1$ is a group of the sub-formulae (a)-(d) as defined in claim 10.

13. A pharmaceutical composition comprising a compound according to any one of claims 1 to 9 or a pro-drug thereof and a pharmaceutically acceptable carrier.

14. A compound according to claim 1 or a pro-drug thereof for use as an anti-inflammatory and/or analgesic agent.

0032314

European Patent Office

# PARTIAL EUROPEAN SEARCH REPORT

which under Rule 45 of the European Patent Convention shall be considered, for the purposes of subsequent proceedings, as the European search report

Application number

EP 80304710.9

## DOCUMENTS CONSIDERED TO BE RELEVANT

| Category | Citation of document with indication, where appropriate, of relevant passages | Relevant to claim | CLASSIFICATION OF THE APPLICATION (Int Cl. ³) |
|---|---|---|---|
| | DE - A1 - 2 819 463 (BEECHAM)<br>+ Claims + | 1-14 | C 07 D 207/333<br>C 07 D 409/06 |
| P | & US-A-4 200 645 (GOUDIE)<br>(29-04-1980)<br>-- | | A 61 K 31/40 |
| | US - A - 3 721 680 (CARSON)<br>+ Abstract; claim 1 +<br>---- | 1 | |

TECHNICAL FIELDS SEARCHED (Int. Cl. ³)

C 07 D 207/00
C 07 D 409/00
A 61 K 31/00

## INCOMPLETE SEARCH

The Search Division considers that the present European patent application does not comply with the provisions of the European Patent Convention to such an extent that it is not possible to carry out a meaningful search into the state of the art on the basis of some of the claims.

Claims searched completely: 9-12

Claims searched incompletely: 1-8,13,14

Claims not searched: -

Reason for the limitation of the search:

In the claims 1-8, 13 and 14 there is no explanation of the term "pro-drug" given.

CATEGORY OF CITED DOCUMENTS

X: particularly relevant
A: technological background
O: non-written disclosure
P: intermediate document
T: theory or principle underlying the invention
E: conflicting application
D: document cited in the application
L: citation for other reasons

&: member of the same patent family, corresponding document

| Place of search | Date of completion of the search | Examiner |
|---|---|---|
| VIENNA | 07-04-1981 | ONDER |

EPO Form 1505.1 06.78